# EUROPEAN PATENT APPLICATION

(11) **EP 4 631 357 A1**
(43) Date of publication of application: **15.10.2025**
(21) Application number: 24305597.7
(22) Date of filing: 12.04.2024
(51) Int. Cl.: A23C 11/10, A23L 11/50, A23L 11/60, A23L 33/135, C12N 1/20, C12R 1/46

(54) **LACTOCOCCUS BACTERIAL STRAINS FOR FERMENTATION OF PLANT-BASED PREPARATION**

(71) Applicant: SAS C&DAC, 54500 Vandoeuvre-lès-Nancy (FR)
(72) Inventor: BOURCIER, Elise, 54500 Vand uvre-lès-Nancy (FR); CÂMARA JUNIOR, Antonio de Anchieta, 54500 Vand uvre-lès-Nancy (FR)
(74) Representative: Icosa

(57) **Abstract**

The present invention relates to bacterial strains belonging to the species *Lactococcus lactis* and further relates to their used the fermentation process when inoculated with a plant-based preparation, for the production of dairy alternative products.

## Description

### FIELD OF INVENTION

The present invention relates to the field of food and/or feed industry. In particular, the present invention relates to the fermentation process of plant-based preparations, and the use of the fermented plant-based products for the production of dairy alternative products. The present invention also relates to bacterial strains belonging to the species *Lactococcus lactis*, and a composition and a method comprising at least one of these bacterial strains for the production of dairy alternative products.

### BACKGROUND OF INVENTION

Industrial fermentation is a large-scale process that is used in the production of food and/or feed products. Fermentation is performed by microorganisms, like bacteria, which are responsible for key transformation enabling desirable characteristics to the final product. Of particular importance during fermentation, bacteria play a pivotal role in acidifying the preparation. This process is very important as it ensures product texture, flavor, nutritional quality and conservation. However, due to society concerns (*e.g.*, carbon transition, conservation, health, and animal welfare), the food and/or feed industry is facing a shift toward the production of dairy alternative products, resulting from the use of fermented plant-based products. This shift thus necessarily involves adapting current production processes methods and protocols.

Acid lactic bacteria, such as *Lactococcus lactis*, are routinely used for industrial fermentation. However, traditional fermentation methods often require extended period to achieve the desired level of acidification, posing challenge in terms of efficiency and production timelines. As reported in WO2023001633, the required time to reach an acidic environment of pH 6.0 lasts of about 6 to 8 hours. This extended fermentation duration not only impacts productivity but also hinders the industry's ability to meet the growing demand for dairy alternative products in a timely manner. Moreover, reduction of the fermentation duration would prevent the development of undesirable microorganisms such as pathogens Thus, there is unmet need to optimize the acidification rate so as to expedite the fermentation, while maintaining product quality.

Besides the biochemical characteristics related to the fermentation process, the raw material is another fundamental aspect that interferes with the quality and the production of dairy alternative products. Among the available plant sources, pulses are particularly interesting, as they are easily cultivable. Moreover, pulses are interesting in agriculture thanks to its nitrogen fixation ability. Furthermore, pulses are very interesting for their intrinsic characteristics such as a high protein content, a high nutrient value (*e.g.*, starch, fibers, vitamins, minerals, etc.), their poor allergen content. Moreover, pulses provide a good texture, flavor, aroma, and sustainability to the final product. Although these interesting properties, some pulses species however provide astringency, mostly due to tannins, and green taste, mostly due to aldehydes, to the final product. There is thus also a need to improve the organoleptic profile of dairy alternative products, in particular plant-based products. Moreover, some pulses species contain antinutritional factors (ANFs) such as phytic acid, antitrypsic factors, vicine/convicine, saponins, raffinose, etc. These ANFs are compounds that tends to reduce the availability of nutrients.

Taken together, there is a growing imperative to discover new bacteria strains with enhanced capabilities and properties so as to improve the production, the quality and sustainability of dairy alternative products.

In the present invention, the Applicant selected for its search the faba bean, for both its protein content, its starch content, its high ability to fix atmospheric nitrogen and its ability to grown with a limited amount of water.

Surprisingly, the Applicant identified two novel bacterial strains, 552-4 and 552-12 belonging to the species *Lactococcus lactis*, from the faba bean ("*Vicia faba*"), that showed to be beneficial strains improving acidification during the fermentation process of plant-based products: the Applicant herein shows that these novel bacterial strains significantly reduce the acidification kinetic of fermentation. When tasting the final product resulting from the use of the fermented plant-based preparation, the Applicant unexpectedly realized that astringency was considerably lower than expected. Green taste, which is an off-note, was hardly perceptible, according to a panel of tasters. On the more, the content in antinutritional factors such as vicine-convicine, trypsin inhibitors, phytates, and raffinose was sensibly reduced.

The present invention thus relates to new bacterial strains belonging to the species *Lactococcus lactis* enabling to improve the fermentation process of plant-based preparation, while enhancing the final product organoleptic profile and quality. These bacterial strains are therefore the solution to help the food and/or feed industry to adapt their protocols for producing dairy alternatives products, in response to consumer demand and current societal concerns.

### SUMMARY

This invention thus relates to a bacterial strain belonging to the species *Lactococcus lactis*, wherein the bacterial strain is selected from strains 552-4 and 552-12, deposited at the CNCM on March 13, 2024 as CNCM 1-6045 and CNCM I-6046, respectively.

In some embodiments, the bacterial strain is isolated from *Vicia faba.*

In some embodiments, the bacterial strain enables the acidification of fermentation when inoculated in a plant-based preparation, wherein the acidification rate has an average time for reaching the pH 6.0 of less than about 300 minutes and an average time for reaching the pH 5.0 of less than about 750 min.

In some embodiments, the plant-based preparation is derived from legumesplants such as faba bean, soya bean, chick pea, lentil and the like; from oilseeds such as colza, sunflower, canola oil, rapeseed, soya bean, sesame, cotton and the like; from cereals or pseudo cereals such as wheat, corn/maize, oats, sorghum, rice, barley, millet, triticale, buckwheat, rye, teff and the like; from nuts such as almond, coconut, cashew nut, Brazil nut, hazelnut, macadamia nut, pecan nut, pistachio and walnut and the like; from tuber such as cassava, potato, tapioca, arrowroot and the like; or from any combination thereof.

The invention further relates to a composition comprising at least one bacterial strain.

In some embodiments, the form of the composition is a liquid, a solid, a semi-liquid (viscous), a frozen or a dried-powder.

In some embodiments, the composition further comprises sugar at an amount of about 1 to 5%.

In some embodiments, the composition further comprises at least one other microorganism selected from bacteria, molds, fungi and/or yeasts species.

The invention further relates to a fermented plant-based product comprising at least one bacterial strain according to the invention or at least one composition according to the invention.

In some embodiments, the plant-based product according further comprises the plant-based preparation according to the invention.

The invention further relates to a dairy alternative product comprising at least one bacterial strain according to the invention, or at least one composition according to the invention, or at least a fermented plant-based product according to the invention, and further comprising any other compounds suitable for the preparation of the dairy alternative product.

In some embodiments, the dairy alternative product has improved organoleptic profile and nutritional values.

The invention further relates to a food and/or feed product comprising at least one bacterial strain according to the invention, or at least one composition according to the invention, or at least a fermented plant-based product according to the invention, or a dairy alternative product according to the invention.

The invention further relates to the use of at least one bacterial strain according to the invention, or at least one composition according to the invention, for the production of a fermented plant-based product according to the invention, or for the production of a dairy alternative product according to the invention, or for the production of a food and/or feed product according to the invention.

The invention further relates to a method for the production of a fermented plant-based product, comprising the step of:
i. preparing a plant-based preparation comprising from about 0.5 to 15% by weight of dissolved plant proteins into water;
ii. inoculating said plant-based solution with bacterial strain according to the invention at a concentration of about 10⁶ to 10⁸ CFU / mL substrate;
iii. fermenting the inoculated plant-based solution obtained at step iii), by incubation at a temperature from about 25 to 39°C for about 0.5 to 24 hours;
iv. obtaining a said fermented plant-based product.

### DEFINITIONS

In the present invention, the following terms have the following meanings:
"**About**" when preceding a figure, encompasses plus or minus 10%, or less, of the value of said figure. It is to be understood that the value to which the term "about" refers is itself also specifically, and preferably, disclosed.
"**At least**" refers to a starting point of an open range of element.
"**Bacterial strain**" refers to a subtype of a bacterial species.
"**CFU**" refers to an abbreviation of the term "colony forming unit"
"**CNCM**" refers to the « *Collection Nationale de Cultures de Microorganismes »* (Institut Paster, 25 rue du Docteur Roux, Paris, France) under the Budapest Treaty. Each microorganism deposited at the CNCM is then referred with an accession number consisting of the CNCM acronym followed by 4 digits numbers (*e.g.*, CNCM-1234). As used herein, the bacterial strains 552-4 and 552-12, deposited at the CNCM on March 13, 2024 have the accession number CNCM 1-6045 and CNCM 1-6046, respectively.
**"Antinutritional factors** or "**ANFs**"" refers to compounds that tends to reduce the availability of nutrients or have adverse effects on digestion. AFNs may be phytic acid, antitrypsic factors, vicine/convicine, saponins, raffinose, and the like. AFNs may be present in food. AFNs may be present in plant species, such as pulses species. When AFNs are low in final food and/or feed products, it is meant that these products have high nutritional values.
**"Feed product"** refers to any substance or mixture that are used as food for animal. These animals include pets like dogs, cats, birds, rodents, fish, and the like, and others domestic animals; for livestock like cattle, poultry, pigs, aquacultures of any fish or crustacea species; for wild animals like animals kept in captivities such as zoo.
**"Fermentation"** refers to the anaerobic metabolic process that consumes sugar in the absence of oxygen. The products are organic acids, gases, or alcohol. It occurs in microorganisms, such as yeast and bacteria and the like, and also in oxygen-starved muscle cells, as in the case of lactic acid fermentation.
**"Fermented plant-based product"** refers to the product arising from the fermentation process, wherein bacteria are inoculated with a plant-based preparation. The fermented plant-based product may be further used for the preparation of final food and/or beverages products (for human), or feed products (for animals), and for dairy alternative products. It is thus meant that "fermented plant-based products" are an intermediary product and/or preparation between the fermentation process and the preparation of the consumer product.
**"Food product"** refers to industrial food production for human consumption. Food product may include food and/or beverage and the like.
**"Dairy alternative product"** refers to the final consumable product comprising or consisting of non-animal matter (*i.e.,* the lacteal secretion obtained by milking). The dairy alternative product may derive from plant sources. The dairy alternative product according to the invention is produced with fermented plant-based products. Dairy alternative products are generally consumed by individuals who are lactose intolerant, allergic to dairy proteins, following a vegan lifestyle, or seeking to reduce their consumption of animal-derived products. Dairy alternative products may be for human (such as a food and/or beverage products). Dairy alternative products may be meat analogue. The term "dairy alternative product" may also be read as "plant-based dairy substitutes", or "dairy analogue product" or "dairy substitute product" and these terms can be used interchangeably. For the sake of clarity, terms "dairy alternative products" are used throughout the text of this application.
**"Off-notes",** also called off-flavor, refers to undesirable, unacceptable or unpleasant sensory characteristics in a food or beverage product. Theses can result from various factors selected in the list comprising ingredient source (*i.e.,* the plant source), ingredient quality, processing methods, storage conditions, or interactions between ingredients. Identifying and mitigating off-notes is one of the most important challenge of food and feed industry, in particular dairy alternative product such as plant-based product, for maintaining the quality and consumer acceptance of food products, as these are common reason for consumer rejection of food/beverage products.
**"Organoleptic profile"** refers to the sensory characteristics of a food, beverage products, water, and the like. These characteristics are selected in the list comprising appearance, flavor, smell, texture, aroma, and mouthfeel, and the like. It involves subjective evaluations by trained professionals or sensory panels so as to assess qualities like sweetness, bitterness, sourness, saltiness, umami, and various nuances within these parameters.
**"Sugar"** refers to chemical compounds made by carbon, hydrogen, and oxygens atoms with the general formula of CₙH2ₙOₙ. The sugar may glucose, sucrose, lactose, maltose, fructose, galactose, and the like. In the context of food, sugar refers to a class of carbohydrates that are sweet-tasting, soluble in water, and typically crystalline in structure.
**"Plant-based preparation"** refers to a preparation that does not comprise animal-derived matter (*e.g.*, mammal milk). According to the invention, the plant-based preparation will be mix with at least one microorganism, preferably at least one bacterium, so as to perform fermentation, thereby generating a fermented plant-based product. As used herein, the terms "plant" or "vegetal" can be used interchangeably. For the sake of clarity, terms "plant" is used throughout the text of this application. As used herein, terms "preparation" or "composition" or "substrate" can be used interchangeably. For the sake of clarity, term "preparation" is used throughout the text of this application.

### DETAILED DESCRIPTION

The Applicant has identified bacterial strains belonging to *Lactococcus* genus, in particular from *Lactococcus lactis* species. These bacterial strains were isolated from the faba bean, a pulse rarely used for the production of dairy alternative products.

In some embodiments, the bacterial strain is from *Lactococcus lactis subsp. hordniae.*

In some embodiments, the bacterial strain provides numerous benefits for the production of dairy alternative products and/or for food and/or feed industry. The benefits may be an improvement of the process (*i.e.,* the fermentation process, etc.) or an improvement of the quality of the final product (*i.e.,* organoleptic profile, nutritional value, etc.). By performing fermentation activity tests, the Applicant has surprisingly found that these bacterial strains consistently speed up the acidification rate occurring during the fermentation process when inoculated in a plant-based preparation. The Applicant thus has identified new bacterial strains improving the fermentation process of plant-based preparations, thereby improving the production of dairy alternative products for food and/or feed industry. When testing the final product, the Applicant unexpectedly realized that the organoleptic profile of the final product was improved. Products testing by consumers has highlighted that undesirable characteristics such as astringency and off-notes were strongly diminished. Moreover, when analyzing the final product composition, antinutritional substances were significantly reduced in the final products. Hence, the newly identified bacterial strains improve the quality of dairy alternative products for food and/or feed industry, both in terms of organoleptic profile and nutritional value.

Throughout the present demand, when terms "bacterial strain" are used, it is meant these terms may refer to either strain 552-4 or strain 552-12 or both strains - (used in consortium).

In some embodiments, the bacterial strain improves the fermentation process when inoculated in any plant-based preparations. In a preferred embodiment, the bacterial strain when inoculated in any plant-based preparations accelerate the acidification rate.

As used herein, fermentation is the metabolic process that converts carbohydrates (*e.g.*, sugar, starches, and the like), into acids, gases or alcohol in the absence of oxygen. This process is carried out by microorganisms, such as bacteria, yeasts, molds, fungi, and the like. Fermentation is used in the field of food industry so as to produce various food and/or feed products. The fermentation is performed on a wide range of raw materials or substrates that may derived from any plant or animal species. During fermentation, microorganisms such as lactic acid bacteria (*e.g., Lactococcus lactis*) or acetic acid bacteria metabolize the carbohydrate present in food and/or beverage substrates or preparations, and produce organic acids (*e.g.*, lactic acid, acetic acid, citric acid, and the like). These organic acids lower the pH of the environment, leading to acidification of the food/beverage preparation. The fermentation thus generates fermented products, which are used in food and/or feed industry so as to prepare final consumable products (e.g., milk, yoghurt, cheese, and the like). As used herein, fermented plant-based products are generated through the fermentation step, so as to produce dairy alternative products for food and/or feed industry.

In some embodiments, the fermentation process of the present invention is a semi-solid-state fermentation in liquid submersion.

In some embodiments, the fermentation process is performed under agitation. In some embodiments, the fermentation process is not performed under agitation.

In some embodiments, the fermentation process is performed with any plant-derived matters. In some embodiments, the fermentation process is performed with any plant-based preparations.

In some embodiments, the fermentation process is performed at a temperature from about 25 to 45 °C. The temperature may be 25, 26, 27, 28, 29, 30, 30,5, 31, 31,5, 32, 32,5, 33, 33,5, 34, 34,5, 35, 35,5, 36, 36,5, 37, 38, 39, 40, 41, 42, 43, 45°C. In a preferred embodiment, the fermentation process is performed at 35°C.

In some embodiments, the fermentation process lasts for about 0.5 to 24 hours. The fermentation process may last for about 0.5 up to 23 or 22 or 21, or 20, or 19, or 18, or 17, or 16, or 15, or 14, or 13, or 12, or 11, or 10, or 9, or 8, or 7, or 6, or 5, or 4, or 3, or 2, or 1 hours.

The present invention first relates to at least one a bacterial strain belonging to the species *Lactococcus lactis*, wherein the at least one bacterial strain is selected from strains 552-4 and/or 552-12, deposited at the CNCM on March 13, 2024 as CNCM I-6045 and CNCM I-6046, respectively.

Bacterial strains according to the invention belonging to the species *Lactococcus lactis* are described for the first time herein by the Applicant. In some embodiments, the bacterial strains have the following characteristics: cells are not pigmented, non-spore-forming, non-motile, Gram-positive; cells are averaging 0.5 to 1.5 micrometers in size; cells have an anaerobic but aerotolerant metabolism; cells are typically cultivated at a mesophilic temperature which range from about 20°C to 45°C; cells are usually cultivated in M17 or MRS media; cells have an beta-galactosidase activity, a tannase activity, an alpha amylase activity, a beta-glucosidase activity; cells assimilate sugar such as lactose, galactose and fructose.

The sequences of the 16S rRNA gene of *Lactococcus lactis* strains 552-4 and 552-12 are SEQ ID NO: 1 and SEQ ID NO: 2, respectively.

The bacterial strains have been deposited at the *"Collection National de Cultures de Microorganismes (CNCM)"* (Institut Paster 25, Rue du Docteur Roux, F-75724 Paris CEDEX 15), in accordance with the Budapest Treaty on the International Recognition of the Deposit of Microorganisms for the Proposes of Patent Procedure. We hereby confirm that the Depositor (SAS C&DAC) of the bacterial strains as described herein has given his unreserved and irrevocable consent to the deposited material being made available to the public. In respect to those designations in which a European Patent is sought, a sample of the deposited microorganism will be made available until the publication of the mention of the grant of the European patent or until the date on which application has been refused or withdrawn or is deemed to be withdrawn, only by the issue of such a sample to an expert nominated by the person requesting the sample, and approved either a) by the Applicant and/or ii) by the European Patent Office, whichever implies (Rule 21 EPC).

In some embodiments, the bacterial strain is a Lactococcus bacterium. In some embodiments, the bacterial strain is a *Lactococcus lactis* bacterium.

In some embodiments, the bacterial strain is not an enterococcus bacterium.

In some embodiments, the bacterial strain is pasteurized. In some embodiments, the bacterial strain is not pasteurized.

In some embodiments, the bacterial strain is isolated from a plant.

In some embodiments, the bacterial strain is isolated from any parts of the plant. The part of the plant may be leaves, seeds, roots, tubers, bulbs, flowers, stems and shoots, sprouts and the like. In a preferred embodiment, the bacterial strain is isolated from seeds.

In some embodiment, the bacterial strain is isolated from legumes, pulses, grains/cereals, nuts/seeds, roots/tubers, sacchariferous plants, oleiferous plants/oilseeds, fibers plants, tobacco plant, and the like.

In a preferred embodiment, the bacterial strain is isolated from pulse.

In a preferred embodiment, the bacterial strain is isolated from faba beans (Latin name *Vicia faba*). Without being bound to any theories, to the past knowledge of the Applicant, the bacterial strain according to the invention are endogenous to *Vicia faba.*

According to the invention, the bacterial strain is isolated from *Vicia faba.*

In some embodiments, the bacterial strain is isolated from seeds of *Vicia faba.*

In some embodiments, the bacterial strain is inoculated within a plant-based preparation.

According to the invention, the bacterial strain is inoculated in a plant-based preparation at a concentration from about 10⁶ to 10⁸ CFU/mL substrate.

In some embodiments, the bacterial strain of the present invention is inoculated in any plant-based preparation at a concentration from about 10⁵ to 10⁹ CFU/mL substrate. The concentration of the bacterial strain may be from about 10⁶ to 10⁹ CFU/mL, or 10⁷ to 10⁹ CFU/mL, or 10⁸ to 10⁹ CFU/mL, or 10⁶ to 10⁸ CFU/mL, or 10⁶ to 10⁷ CFU/mL substrate. In some embodiments, the concentration of the bacterial strain is from about 10⁶ to 10⁸ CFU/mL substrate. In a preferred embodiment, the bacterial strain of the present invention is inoculated in any plant-based preparation at a concentration of 10⁷ CFU/mL substrate.

In some embodiments, the plant-based preparation according to the invention does not comprise animal-derived matter (*e.g.*, mammal milk). In some embodiments, the plant-based preparation comprises animal-derived matter (*e.g.*, mammal milk).

According to the invention, the plant-based preparation is derived from legumes such as faba beans, soybeans, black beans, mung beans, lima bean, chickpeas, lentil, lupin, and the like; from oilseeds such as colza, sunflower, canola oil, rapeseed, soya bean, sesame, chia, flax, hemp, and the like; from cereals or pseudo-cereals such as wheat, corn/maize, oats, hemp, sorghum, rice, barley, millet, triticale, buckwheat, rye, teff and the like; from nuts such as almond, coconut, cashew nut, Brazil nut, hazelnut, macadamia nut, pecan nut, pistachio and walnut and the like; from tuber such as cassava, potato, tapioca, arrowroot and the like; and/or from any combinations thereof.

In some embodiments, the plant-based preparation is derived from legumes. In some embodiments, the plant-based preparation is derived from pulses.

In some embodiments, the plant-based preparation is derived from faba bean, soy, pea, chickpea, lentil, lupin and the like. In a preferred embodiment, the plant-based preparation is derived from faba bean.

In some embodiments, the plant-based preparation is derived from soy. In some embodiments, the plant-based preparation is not derived from soy.

In some embodiments, the plant-based preparation is derived from any parts of the plant. The part of the plant may be leaves, seeds, roots, tubers, bulbs, flowers, stems and shoots, sprouts and the like. In a preferred embodiment, the plant-based preparation is derived from seeds.

In a certain embodiment, the plant-based preparation is derived from bean seeds. In a preferred embodiment, the plant-based preparation is isolated from faba bean seeds (*Vicia faba* seeds).

In some embodiments, the seeds are resuspended into water for the preparation of a plant-based preparation. It is meant that the plant-based preparation is an aqueous suspension. In some embodiment, the aqueous solution resulting from the mix of the plant substrate and water is termed "juice".

In some embodiments, the ratio seeds and water is 1:3. In some embodiments, the ratio seeds and water is 1:2.

In some embodiments, the seeds are pre-treated prior being resuspended into water for the preparation of the plant-based preparation. The seeds may be cleaned, hulled, crushed, soaked, dried, or any combination thereof. In some embodiments, the seeds pretreatment may further comprise a mechanical transformation into flour. It is to be understood that the pretreatment of the seeds may be adapted according to the plant origin/species of the seeds and/or seeds morphological characteristics.

In some embodiments, the plant-based preparation in an aqueous suspension comprises from about 0,5 to 15% by weight of dissolved plant proteins. The plant-based preparation in an aqueous suspension may be from about 2 to 12%, or about 4 to 10%, or about 6 to 8% by weight of dissolved plant proteins. In a preferred embodiment, the plant-based preparation in an aqueous suspension comprises 8% by weight of dissolved plant proteins.

According to the invention, the plant-based preparation in an aqueous suspension comprising from about 0.5 to 15% by weight of dissolved plant proteins.

In some embodiments, the plant-based preparation is homogenous. In some embodiments, the plant-based preparation comprises one type of plant substrate or matter.

In some embodiments, the plant-based preparation is heterogenous. In some embodiments, the plant-based preparation comprises at least one other type of plant-derived matter.

In some embodiments, the plant-based preparation is a mix of proteins extracted from different plant species. In some embodiments, the mix of proteins extracted from different plant species may be in equal or non-equal proportions.

In some embodiments, the bacterial strain acidifies the plant-based preparation to a pH less than about pH 7.0. In some embodiments, the bacterial strain acidifies the plant-based preparation to a pH value from about pH 7.0 to 1.0, preferably from about pH 7.0 to 3.5. The pH value may be pH 6.9, 6.8, 6.7, 6.6, 6.5, 6.4, 6.3, 6.2, 6.1, 6.0, 5.9, 5.8, 5.7, 5.6, 5.5, 5.4, 5.3, 5.2, 5.1, 5.0, 4.9, 4.8, 4.7, 4.6, 4.5, 4.4, 4.3, 4.2, 4.1, 4.0, 3.9, 3.8, 3.7, 3.6, 3.5.

In a preferred embodiment, the bacterial strain acidifies the plant-based preparation to a pH value less than about pH 6.0. In a preferred embodiment, the bacterial strain acidifies the plant-based preparation to a pH value less than about pH 5.0.

In some embodiments, the acidification rate for reaching pH 6.0 is less than about 500 min. The acidification rate may be less than about 500, 490, 480, 470, 460, 450, 440, 430, 420, 410, 400, 390, 380, 370, 360, 350, 340, 330, 320, 310, 300, 290, 280, 270, 260, 250, 240, 230, 220, 210, 200, 190, 180, 170, 160, 150, 140, 130, 120, 110, 100, 90, 80, 70, 60, 50, 40 or 30 min.

In some embodiments, the acidification rate for reaching pH 6.0 is from about 180 to 60 min.

In some embodiments, the acidification rate for reaching pH 5.0 may be less than about 800 min. The acidification rate may be less than about 800, 790, 780, 770, 760, 750, 740, 730, 720, 710, 700, 690, 680, 670, 660, 650, 640, 630, 620, 610, 600, 590, 580, 570, 560, 550, 540, 530, 520, 510, 500, 490, 480, 470, 460, 450, 440, 430, 420, 410, 400, 390, 380, 370, 360, 350, 340, 330, 320, 310, 300, 290, 280, 270, 260, 250, 240, 230, 220, 210, 200, 190, 180, 170, 160, 150, 140, 130, 120, 110, 100, 90, 80, 70 or 60 min.

In a certain embodiment, the acidification rate for reaching pH 5.0 is from about 720 to 600 min.

According to the invention, the bacterial strain enables the acidification of fermentation when inoculated in a plant-based preparation, wherein the acidification rate has an average time for reaching the pH 6.0 of less than about 300 minutes and an average time for reaching the pH 5.0 of less than about 750 min.

In some embodiments, the acidification rate is faster with the bacterial strain 552-4 than with the bacterial strain 552-12.

In some embodiments, the acidification rate for reaching pH 6.0 may be less than 120 min when using the bacterial strain 552-4. In some embodiments, the acidification rate for reaching pH 5.0 may be less than 300 min when using the bacterial strain 552-4.

In some embodiments, the bacteria strain has a maximum specific growth rate when inoculated in the plant-based preparation from about 2 hours. The maximum specific growth rate may be less than about 2.0, 1.9, 1.8, 1.7, 1.6, 1.5, 1.4, 1.3, 1.2, 1.0, 0.9, 0.8, 0.7, 0.6, 0.5, 0.4, 0.3, 0.2 or 0.1 hours.

In some embodiments, the bacterial strain improves the organoleptic profile of any food and/or feed products. In some embodiments, the bacterial strain improves the organoleptic profile of dairy alternative products.

In some embodiments, the bacterial strain improves the nutritional value of any food and/or feed products. In some embodiments, the bacterial strain improves the organoleptic profile of dairy alternative products.

In some embodiments, the bacterial strain synthetizes enzymes interfering with the fermentation process of any plant-based preparation. In some embodiments, the bacterial strain synthetizes enzymes improving the quality of the fermented plant-based products.

In some embodiments, the bacterial strain has an alpha-galactosidase activity. It is meant that an alpha-galactosidase activity enables the degradation of oligosaccharides, such as raffinose and the like, that are not desirable in any final products, preferably dairy alternative products.

In some embodiments, the bacterial strain has a beta-glucosidase activity. It is meant that a beta-glucosidase activity enables the degradation of substances comprising glycosidic bonds, such as vicin or convicin and the like, that are not desirable in any final products, preferably dairy alternative products.

In some embodiments, the bacterial strain has a tannase activity. It is meant that tannase activity enables to break down hydrolysable tannins, thereby reducing the astringent taste. In some embodiments, the bacterial strain reduces the astringency of any final products, preferably dairy alternative products.

In some embodiments, the bacterial strain has an alpha-amylase activity. It is meant that an alpha-amylase activity enables the degradation of starch, which mainly participates in food texture and digestibility, that could be not desirable in final products, preferably dairy alternative products.

In some embodiments, the bacterial strain contributes to the quality of the fermented product, preferably the fermented plant-based product. The benefits may be improving the organoleptic profile such as the taste profile (*e.g.,* flavor, aroma, etc.) and the texture (e.g., mouthfeel and consistency) and the like; enabling the bioavailability of nutrients, improving the digestibility (e.g., by reducing antinutrients), improving the preservation of the final consumable product.

In some embodiments, the bacterial strain enhances the emulsifying activity of the fermented-based product.

In some embodiments, the bacterial strain enhances the absorption capacity of the fermented-based product.

In some embodiments, the fermented plant-based product is in various form. The fermented plant-based product may be granules, pellets, flour, paste, liquids, sauce, condiments, and the like.

In some embodiments, the fermented plant-based product is for the production of food and/or feed products, preferably for the production of dairy alternative products.

In some embodiments, the dairy alternative products are selected in the list comprising milk alternative products, yogurt alternative products, pudding alternative products, cheese alternative products, butter alternative products, cream alterative products, sour cream alternative products, ice cream alternative products, meat alternative/analogue products, biscuits, cookies, breads, and the like.

The invention further relates to a composition comprising at least one bacterial strain, as described herein above.

In some embodiments, the composition comprises at least the bacterial strains 552-4 and 552-12. In some embodiments, the composition comprises at least one bacterial strain among bacterial strains 552-4 or 552-12. In some embodiments, the composition comprises at least the bacterial strain 552-4, but not 552-12. In some embodiments, the composition comprises at least the bacterial strain 552-12, but not 552-4.

In some embodiments, the composition further comprises any plant-based preparations, as describe herein above.

According to the invention, the form of the composition is a liquid, a semi-liquid, a frozen or a dried-powder.

In some embodiment, the solid form of the composition is obtained by drying, dehydration or lyophilization.

In some embodiments, the composition further comprises sugar. The sugar may glucose, sucrose, lactose, maltose, trehalose, fructose, galactose, and the like, or any combination thereof.

In some embodiments, the composition further comprising sugar at an amount of about 1 to 30%. In some embodiments, the composition further comprise sugar at an amount selected of about 1 to 30%, or 1 to 20%, or 1 to 10 %. In some embodiments, the composition further comprise sugar at an amount selected of about 1 to 9%, or 1 to 8% or 1 to 7% or 1 to 6%, 1 to 5%. In some embodiments, the composition further comprises sugar at an amount of about 1, or 2, or 3, or 4 or 5%. In a preferred embodiment, the composition further comprises sugar at an amount of 2%.

According to the invention, the composition further comprises sugar at an amount of about 1 to 5%.

In some embodiments, the composition further comprises food acceptable compounds. By compounds, it is meant a molecule or a solution which is not a microorganism.

In some embodiments, the composition further comprises any food acceptable compounds. The food acceptable compounds may be cryoprotective agents, boosters, additives, and the like, or any combination thereof.

In some embodiments, the cryoprotective agents may be cyclodextrin, maltitol, trehalose, sucrose, maltodextrine, glycerol and/or any combinations thereof.

In some embodiments, the boosters may be nucleotides.

In some embodiments, additives may be added.

In some embodiments, the composition further comprises at least one other microorganism.

In some embodiments, the composition comprises at least one other microorganisms, in equal or non-equal proportions.

According to the invention, the composition further comprise at least one other microorganism selected from bacteria, molds, fungi and/or yeasts species.

In some embodiments, the at least one other microorganism is a bacterium. The bacterium may be from *Lactococcus* species, *Bifidobacterium* species, *Brevibacterium* species, *Proprionibacterium* species, or any combination thereof.

In some embodiments, the at least one other microorganism is another lactic acid bacterium. The lactic acid bacterium may be from *Lactococcus* species, *Streptococcus* species, *Lactobacillus* species, *Enterococcus* species, *Pediococcus* species, *Leuconostoc* species, *Oenococcus* species, or any combination thereof.

In some embodiments, the at least one other microorganism is another lactic acid bacterium. The lactic acid bacterium may be *Lactobacillus acidophilus, Lacticaseibacillus casei, Lactiplantibacillus plantarum, Lacticaseibacillusrhamnosus, Lactobacillus helveticus, Lactobacillus delbrueckii subsp. bulgaricus, Limosilactobacillus fermentum, Limosilactobacillus reuteri, Lactococcus lactis, Lactococcus cremosis, Streptococcus thermophilus,* and the like.

According to the invention, the at least one other microorganism is another lactic acid bacterium selected from *Lactococcus lactis, Lactococcus cremoris,* or *Streptoccocus thermophilus*, or *Lactobacillus delbrueckii subsp. Bulgaricus*, and/or any combination thereof.

In some embodiments, the composition is for the production of a fermented plant-based product.

In some embodiments, the composition is for the production of food and/or feed products, preferably dairy alternative products.

In some embodiments, the composition improves the organoleptic profile of the food and/or feed products. In some embodiments, the composition improves the organoleptic profile of the dairy alternative products.

In some embodiments, the composition improves the nutritional value of food and/or feed products. In some embodiments, the composition improves the nutritional value of the dairy alternative products.

The invention further relates to a fermented plant-based product comprising at least one bacterial strain as describe herein above, or at least one composition as described herein above.

In some embodiment, the fermented plant-based product further comprises a vegetable-based preparation.

The invention further relates to a dairy alternative product comprising at least one bacterial strain as described herein above, or at least one composition as described herein above, or at least a fermented plant-based product as described herein above, and further comprising any other compounds suitable for the preparation of the dairy alternative product.

In some embodiments, the compounds suitable for the preparation of the dairy alternative product may be water, sugar, salt, oil, flavors, aroma, aromatic substances, fruits preparations, starch, cacao, gums, seaweed extracts, and the like.

The invention further relates to a dairy alternative product, wherein the dairy alternative product has improved organoleptic profile and nutritional values.

The invention further relates to a food and/or feed product comprising at least one bacterial strain as described herein above, or at least one composition as described herein above, or at least a fermented plant-based product as described herein above, or a dairy alternative product as described herein above.

The invention also relates to the use of at least one bacterial strain as described herein above, or at least one composition as described herein above, for the production of a fermented plant-based product as described herein above, or for the production of a dairy alternative product as described herein above, or for the production of a food and/or feed product as described herein above.

The invention further relates to a method for the production of a fermented plant-based product, comprising the step of:
i. preparing a plant-based preparation comprising from about 0.5 to 15% by weight of dissolved plant proteins into water;
ii. inoculating said plant-based solution with bacterial strain according to the bacteria of the invention as describe herein above at a concentration of about 10⁶ to 10⁸ CFU / mL substrate;
iii. fermenting the inoculated plant-based solution obtained at step iii), by incubation at a temperature from about 25 to 39^{°}C for about 0.5 to 24 hours;
iv. obtaining a said fermented plant-based product.

In some embodiments, the methods further comprise a step wherein the plant-based preparation is pasteurized.

In some embodiments, the method is for the production of a fermented plant-based product is for the production of a food and/or feed products. In some embodiments, the method is for the production of a fermented plant-based product is for the production of a dairy alternative products.

### BRIEF DESCRIPTION OF THE DRAWINGS

**Figures 1** are line graphs showing the evolution of bacterial growth of *L. lactis* strains 552-2, 552-4, 552-9, 552-12 et 552-15 in M17 medium at 25°C. The bacterial growth is measured by optical density at 600 nm.

**Figures 2** are line graphs showing the evolution of bacterial growth of *L. lactis* strains 552-2, 552-4, 552-9, 552-12 et 552-15 in faba bean juice at 37°C. The bacterial growth is measured by optical density at 600 nm.
**Figures 3 to 5** are line graphs showing the impact of the growth state of bacterial strains 552-4 and 552-12 on the acidification kinetics of fava bean preparation. Both stationary and exponential state of bacterial strain 552-4 and 552-12 are tested. Each line represents a bacterial strain starter culture.
**Figure 6** is a line graph showing the effect of glucose addition on the acidification kinetics of fava bean seeds by the bacterial consortium 552-4/552-12.
**Figure 7** is a line graph showing the acidification kinetics of faba bean preparation as a function of the growth state of bacterial strains 552-4 and 552-12 or the consortium 552-4/552-12, supplemented or not with glucose. Each line represents a bacterial strain(s) starter culture.
**Figure 8** is a bar chart showing raffinose content in faba bean seeds soaked at 4°C or fermented at 25°C for 18h.
**Figures 9 to 11** are radar chart showing the descriptive sensory attributes of plant-based milk formulated with faba bean fermented seeds.
**Figure 12** is a bar chart showing the effect of process duration on the emulsifying activity of fermented faba bean flours prepared with strain 552-4 or 552-12.
**Figure 13** is a bar chart showing the effect of process duration on the water absorption capacity of fermented faba bean flours prepared with strain 552-4 or 552-12.

### EXAMPLES

The present invention is further illustrated by the following examples.

### Example 1: Bacterial growth of strains 552-4 and 552-12 in a faba bean-based preparation

### Materials and Methods

A pre-culture of each of *L. lactis* strains 552-2, 552-4, 552-9, 552-12, 552-15 was performed the previous day at 37°C. The following day, the cultures were plated at the same optical density (OD = 0.620). On a sterile 96-well microplate, 200 µL of M17 media or faba bean juice were seeded with 20 µL of bacterial suspension. Faba bean juice is meant to be the liquid phase resulting from mixing water and crushed faba bean seeds in a 1:3 (w/w) ratio for 30 min.

Absorbance readings at OD 600 nm were taken using a microplate reader (FluoStar Omega BMG LABTECH) for 24 hours at 25°C without agitation. A reading was taken every hour.

### Results

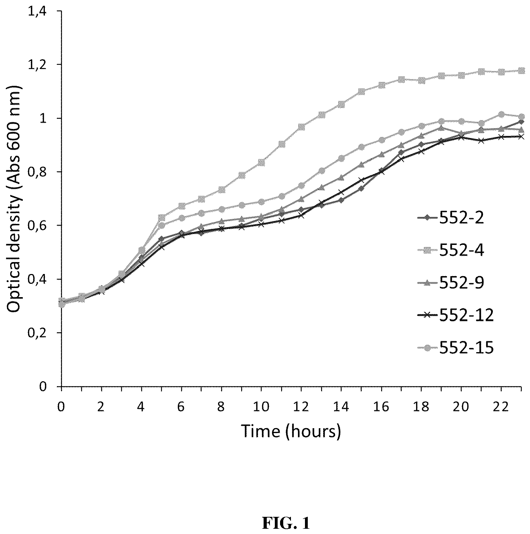

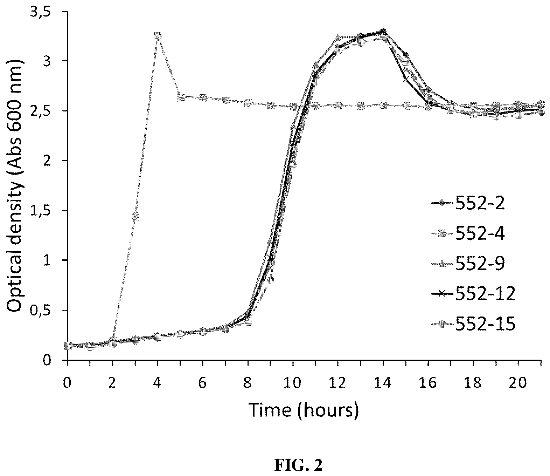

Strain 552-4 shows a growth advantage when placed in a medium limiting fast sugar such as glucose (M17 and faba bean juice) and at 25°C (**Fig. 1**). This advantage of strain 552-4 is much more pronounced in faba bean juice, which is about 6 h ahead of the other strains. All strains grow faster at 37°C than at 25°C, and at higher OD levels. Strain 552-4 showed a faster growth rate than the other strains (**Fig. 1** **and** **Fig. 2**).

### Example 2: Acidification rate of bacterial strains 552-4 and 552-12 in a faba bean preparation

### Materials and Methods

*L. lactis* strains 552-4 and 552-12 were grown for 24 hours at 35°C in MRS medium. Cells were recovered by centrifugation at 3000xg for 5 min and the cell pellet washed twice with sterile physiological 0.9% NaCl solution, then diluted to the same initial volume. The precultures were normalized to the same optical density using a spectrophotometer (OD600nm = 0.005).

The faba bean seeds were transferred to sterile flasks and mixed with sterile distilled water in a 1:3 or 1:2 ratio (for the tests with the bacterial consortium). The inoculation rate was set at 10⁷ CFU/mL of suspension.

Fermentation was carried out without agitation at 35°C in a water bath and pH was monitored continuously using the iCinac system (AMS Alliance, France). Sterile pH probes were inserted aseptically into the flasks and the software was set up to measure pH every 5 min for 18h.

For the tests with the bacterial consortium, with or without the addition of 2% glucose (w/v), the two strains were combined in equal proportions before inoculation and the inoculation rate was the same as the standard procedure.

### Results

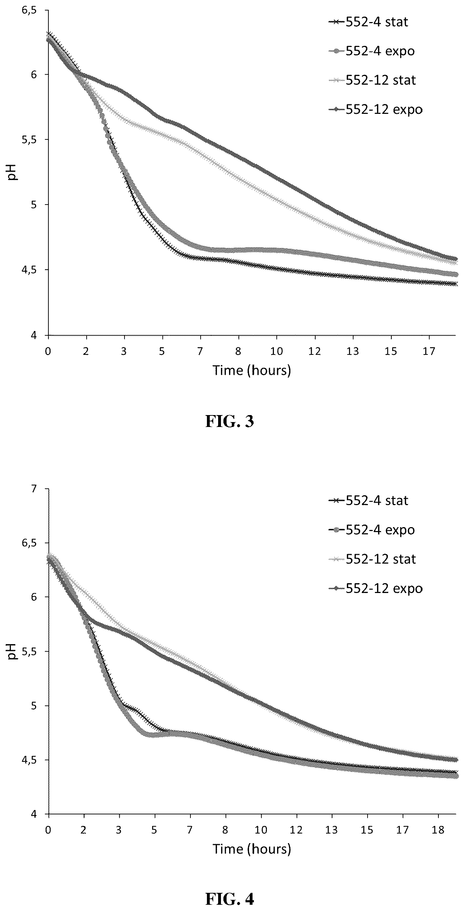

Results showed that both bacterial strains 552-4 and 552-12, when inoculated alone, efficiently acidify the faba bean-based preparation. The time to reach pH 6.0 lasts for 2 hours with both strains, while pH 5.0 is reached in less than 5 and 12 hours with strains 552-4 and 552-12, respectively. Strain 552-4 thus showed a much faster acidification rate than strain 552-12 (**Fig. 3 to 5**).

The physiological state of the two strains 552-4 or 552-12, in stationary or exponential phase, had no significant effect on the acidification kinetics of faba bean-based preparation.

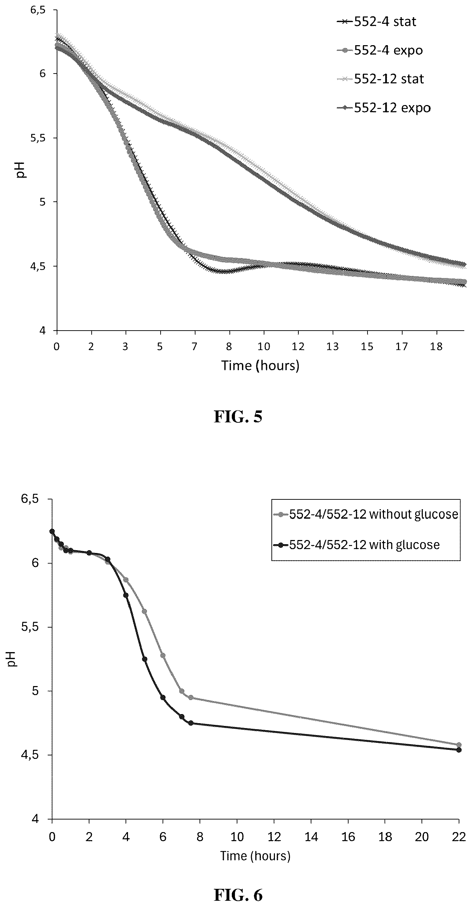

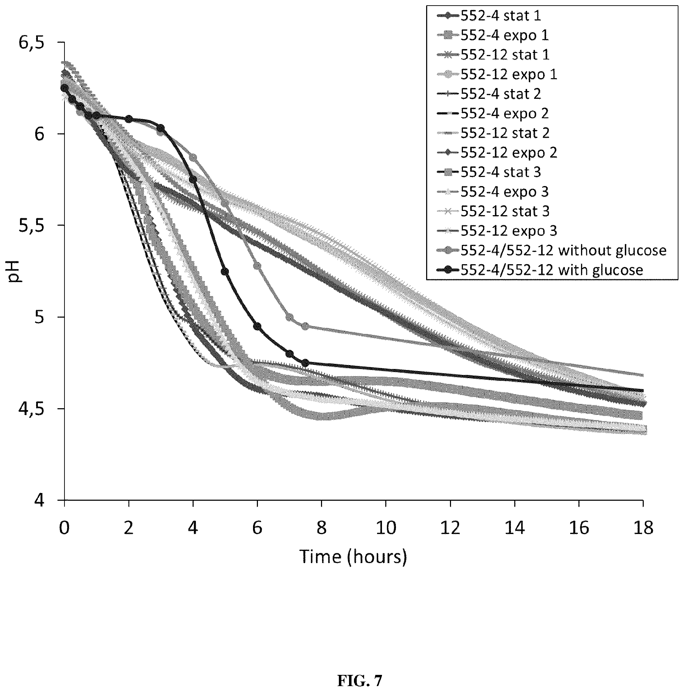

The combination of two strains resulted in faster acidification kinetics than strain 552-12 alone, but less than strain 552-4. Indeed, when combined, bacterial strains 552-4 and 552-12, acidify the faba bean-based preparation to pH 6.0 in less than 4 hours, and while pH 5.0 is reached in less than 10 hours. The addition of 2% glucose to the medium had little effect on the acidification kinetics of the consortium (**Fig. 6** **and** **7**).

These results demonstrate that bacterial strains 552-4 and 552-12, alone or in combination, are very efficient for faba bean acidification.

### Example 3: Antinutritional factors (ANFs) of faba bean preparation fermented with bacterial strains 552-4 and 552-12

### Materials and Methods

*L. lactis* strains 552-2, 552-4, 552-9, 552-12, 552-15 were used for the vicine-convicine, antitryptic factors and phytic acid experiments. *L. lactis* strains 552-2 and 552-4 were used for the raffinose experiments.

For the vicine-convicine experiments, raw (unfermented), soaked or fermented faba bean seeds were analyzed. The mixture of water and crushed faba bean seeds was in a 1:3 ratio. Analyses were carried out by liquid chromatography (HPLC) by the Biogeves laboratory, using an in-house method from Quemener (1988).

For the antitrypsic factors experiments, unfermented, 4°C-soaked or fermented faba bean seeds were analyzed. The mixture of water and crushed faba bean seeds was in a ratio of 1:3 (m/m). Analyses were carried out using the AOCS Ba12-75 method (AOCS, 2011) by the Biogeves laboratory.

For the phytic acid experiments, raw, soaked or fermented faba bean seeds were analyzed. The mixture of water and crushed faba bean seeds was in a ratio of 1:3 (m/m). Analyses were carried out by spectrophotometric assay by the Biogeves laboratory, according to the supplier's recommendations (K-PHYT, Megazyme International, Wicklow, Ireland).

For the raffinose analysis experiments, raw, soaked or fermented, dried and mechanically ground faba bean seeds were analyzed. For soaking and fermentation, the mixture of water and crushed faba bean seeds was in a 1:3 ratio. Analyses were carried out in-house using a spectrophotometric method (K-RAFGL, Megazyme International, Wicklow, Ireland).

### Results

Fermentation significantly reduces the vicin-convicin content of faba bean seeds. The reduction rate is in the order of 40 to 46%, with no remarkable differences between the strains in the Applicant collection. This reduction rate is higher than that of the LC commercial strain (Bioprox). Soaking only reduced vicin-convicin content by 9% (see Table 1 and 2).

**Table 1. Vicine and convicine content in faba bean seeds fermented at 25°C for 17 - 22 hours depending on the strains.**

| **Identifier** | **Sample** | **Vicine-convicine (g/100g MS)** | **Reduction rate*** |
|---|---|---|---|
| 5520 | Non-fermented | 1,03 | - |
| 5522 | Fermented with 552-2 | 0,56 | 46% |
| 5524 | Fermented with 552-4 | 0,59 | 43% |
| 5529 | Fermented with 552-9 | 0,60 | 42% |
| 55212 | Fermented with 552-12 | 0,56 | 46% |
| 55215 | Fermented with 552-15 | 0,60 | 42% |
| 55216 | Fermented with 163-3 (beta-glu negative strain) | 0,65 | 37% |
| 55217 | Fermented with LC (Bioprox) | 0,69 | 33% |

| | | | |
|---|---|---|---|
| *Calculation based on non-fermented sample. | | | |

**Table 2. Vicine and convicine content in fava bean seeds soaked at 4°C or fermented by 552-12 at 37°C for 12 hours.**

| **Identifier** | **Sample** | **Vicine-convicine (g/100g MS)** | **Reduction rate**** |
|---|---|---|---|
| 6870 | Non-fermented | 0,80 | - |
| 2321 | Soaked seeds at 4°C | 0,73 | 9% |
| 6371 | Fermented with 552-12 | 0,48 | 40% |

| | | | |
|---|---|---|---|
| **Calculation based on non-fermented sample. | | | |

Antitrypsin factors are trypsin-inhibiting proteins, so they reduce protein digestibility. Results showed that the rate of reduction of antitrypsin factors by strain 552-12 was 21% higher than that of other strains. However, soaking also had a significant effect on the reduction of antitrypsin factors in faba bean seeds (see Table 3).

**Table 3. Content of trypsin inhibitors in fava bean seeds soaked at 4°C or fermented at 37°C for 12 hours.**

| **Identifier** | **Sample** | **Trypsin inhibitors (TUI/g de MTQ)** | **Reduction rate*** |
|---|---|---|---|
| 5520 | Non-fermented | 5050 | - |
| 5521 | Soaked seeds at 4°C | 3884 | 23% |
| 5522 | Fermented with 552-2 | 5063 | 0% |
| 5524 | Fermented with 552-4 | 5060 | 0% |
| 5529 | Fermented with 552-9 | 4388 | 13% |
| 55212 | Fermented with 552-12 | 3988 | 21% |
| 55215 | Fermented with 552-15 | 4175 | 17% |
| 55216 | Fermented with 163-3 | 4225 | 16% |
| 55217 | Fermented with LC (Bioprox) | 4313 | 15% |

| | | | |
|---|---|---|---|
| *Calculation based on non-fermented sample. | | | |

Phytates are AFNs that reduce the bioavailability of minerals (Fe, Zn, Mg, Ca). Results showed that phytate reduction by the Applicant strains is in the order of 7-15% higher than the commercial LC strain (Bioprox). However, soaking has a more significant effect on phytate reduction (see Table 4).

**Table 4. Phytate content in fava bean seeds soaked at 4°C or fermented at 37°C for 12 hours.**

| **Identifier** | **Sample** | **Phytates (g/100g MTQ)** | **Reduction rate*** |
|---|---|---|---|
| 5520 | Non-fermented | 0.99 | |
| 5521 | Soaked seeds at 4°C | 0.79 | 21% |
| 5522 | Fermented with 552-2 | 0.88 | 12% |
| 5524 | Fermented with 552-4 | 0.89 | 11% |
| 5529 | Fermented with 552-9 | 0.85 | 15% |
| 55212 | Fermented with 552-12 | 0.90 | 10% |
| 55215 | Fermented with 552-15 | 0.90 | 10% |
| 55216 | Fermented with 163-3 | 0.93 | 7% |
| 55217 | Fermented with LC (Bioprox) | 0.95 | 5% |

| | | | |
|---|---|---|---|
| *Calculation based on non-fermented sample. | | | |

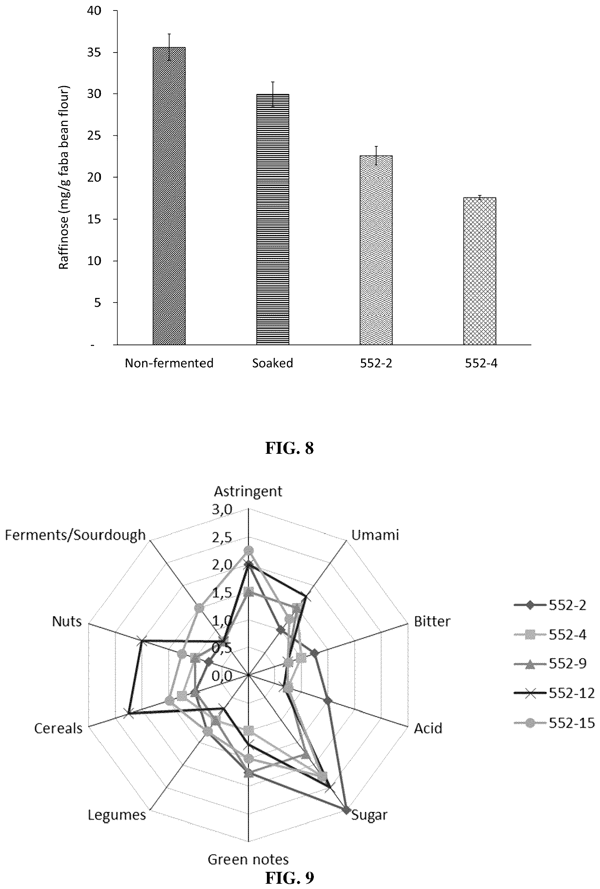

Results from the raffinose experiments showed that fermentation significantly reduces the raffinose content of faba bean seeds. The reduction rate is in the order of 37% (552-2) to 51% (552-4). Soaking only reduces raffinose content by 16% (**Fig. 8**).

### Example 4: Organoleptic profile of faba bean preparation fermented with bacterial strains 552-4 and 552-12

### Materials and Methods

*L. lactis* strains 552-2, 552-4, 552-9, 552-12, 552-15 were inoculated in faba bean preparation.

Vegetable milks produced from fermented faba bean seeds were used. Vegetable milks were prepared by dispersing fermented flours in an aqueous solution containing sugar, neutral oil and water.

The sensory analysis of plant milks was carried out using a hedonic test, the aim of which was to compare products based on panelists' preferences (Thomas, 2016). This test can be carried out using sight and/or by tasting the product. Panelists were asked to rank their preferences on a scale from 0 to 5, with 0 being the lowest score in relation to the attribute being evaluated and 5 the highest.

### Results

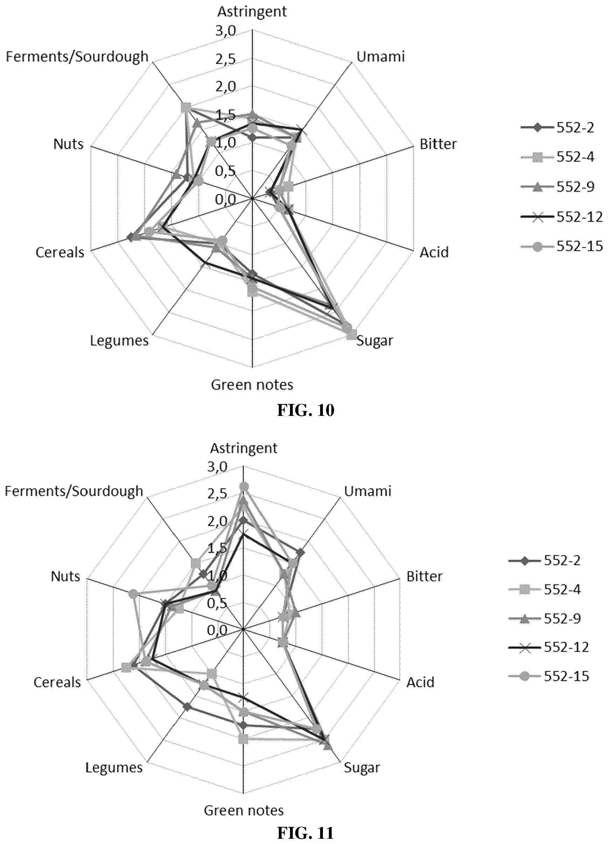

Samples fermented with strains 552-4 and 552-12 have more interesting organoleptic properties than the others, i.e. weaker green notes for 552-4 and more intense cereal and nut notes for 552-12. The milks produced with the flours fermented by 552-12 were chosen as preferred by half the panelists, a trend followed by 552-4 (**Fig. 9 to 11**).

### Example 5: Emulsifying activity of faba bean preparation fermented with bacterial strains 552-4 and 552-12

### Materials and Methods

Faba bean seed meals fermented with strains 552-4 or 552-12 were used. Fermented samples correspond to crushed faba bean seeds mixed with water in a 1:3 ratio.

Emulsifying activity was assessed using the method described by Kaur & Singh (2005). Flours were resuspended in a mixture of water and oil and vigorously mixed. Emulsifying activity was calculated by direct measurement of emulsion expansion before and after treatment.

### Results

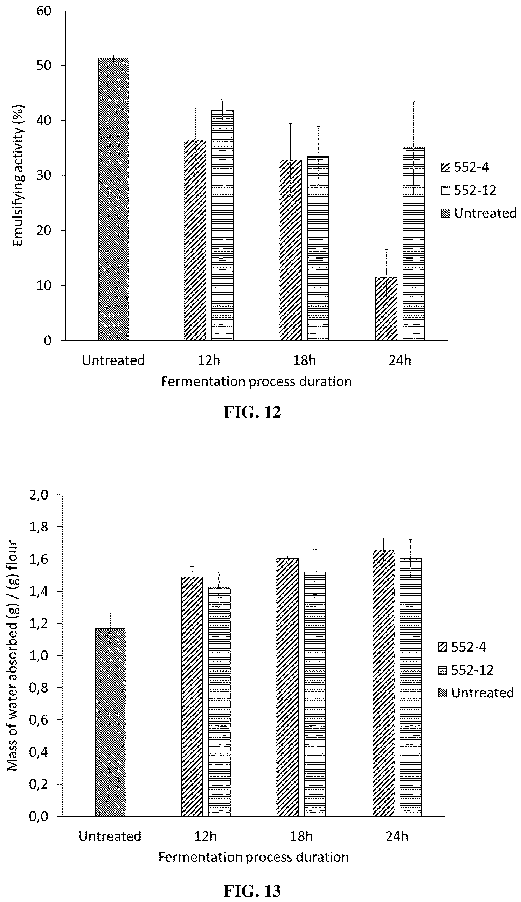

Fermentation affects emulsifying activity. Results showed that a sharp reduction in emulsifying activity was observed for samples fermented with strain 552-4 and after 24h of fermentation. This phenomenon was much less intense for strain 552-12 (**Fig. 12**).

### Example 6: Water retention capacity of faba bean preparation fermented with bacterial strains 552-4 and 552-12

### Materials and Methods

Faba bean seed flours fermented with strains 552-4 or 552-12 were used. Crushed faba bean seeds were mixed with water in a 1:3 ratio.

Water retention capacity was measured using a conventional gravimetric method, which involves dispersing the sample in water and weighing the amount of water absorbed. The solid phase is recovered by centrifugation and the supernatant discarded. The water absorption rate is calculated by comparing the sample weight before and after treatment.

### Results

Water absorption capacity is slightly affected by fermentation, with a higher absorption rate for both strains than for the control. However, this property may also be affected by the drying step during flour preparation (**Fig. 13**).

## Claims

1. A bacterial strain belonging to the species *Lactococcus lactis*, wherein said bacterial strain is selected from strains 552-4 and 552-12, deposited at the CNCM on March 13, 2024 as CNCM I-6045 and CNCM I-6046, respectively.

2. The bacterial strain according to any one of claim **1**, wherein said bacterial strain is isolated from *Vicia faba.*

3. The bacterial strain according to any one of claims **1** to **2**, wherein said bacterial strain enables the acidification of fermentation when inoculated in a plant-based preparation, wherein the acidification rate has an average time for reaching the pH 6.0 of less than about 300 minutes and an average time for reaching the pH 5.0 of less than about 750 min.

4. The bacterial strain according to any one of claims **1** to **3**, wherein the plant-based preparation is derived from legumes plants such as faba bean, soya bean, chick pea, lentil and the like; from oilseeds such as colza, sunflower, canola oil, rapeseed, soya bean, sesame, cotton and the like; from cereals or pseudo cereals such as wheat, corn/maize, oats, sorghum, rice, barley, millet, triticale, buckwheat, rye, teff and the like; from nuts such as almond, coconut, cashew nut, Brazil nut, hazelnut, macadamia nut, pecan nut, pistachio and walnut and the like; from tuber such as cassava, potato, tapioca, arrowroot and the like; or from any combination thereof.

5. A composition comprising at least one bacterial strain according to any one of claims **1** to **4.**

6. The composition according to claim **5**, wherein the form of the composition is a liquid, a frozen or a dried-powder.

7. The composition according to any one of claims **5** or **6**, further comprising sugar at an amount of about 1 to 5%.

8. The composition according to any one of claims **5** to **7** further comprising at least one other microorganism selected from bacteria, molds, fungi and/or yeasts species.

9. A fermented plant-based product comprising at least one bacterial strain according to any one claims **1** to **4** or at least one composition according to any one of claims **5** to **8.**

10. The fermented plant-based product according to claim **9**, further comprising the vegetable-based preparation.

11. A dairy alternative product comprising at least one bacterial strain according to any one claims **1** to **4**, or at least one composition according to any one of claims **5** to **8**, or at least a fermented plant-based product according to any one of claims **9** or **10**, and further comprising any other compounds suitable for the preparation of the dairy alternative product.

12. The dairy alternative product according to claim **11**, wherein said dairy alternative product has improved organoleptic profile and nutritional values.

13. A food and/or feed product comprising at least one bacterial strain according to any one claims **1** to **4**, or at least one composition according to any one of claims **5** to **8**, or at least a fermented plant-based product according to any one of claims **9** or **10**, or a dairy alternative product according to any one of claims **11** or **12.**

14. Use of at least one bacterial strain according to any one of claims **1** to **4**, or at least one composition according to any one claims **5** to **8**, for the production of a fermented plant-based product according to any one of claims **9** or **10**, or for the production of a dairy alternative product according to any one of claims **9** or **10**, or for the production of a food and/or feed product according to claim **13.**

15. A method for the production of a fermented plant-based product, comprising the step of:
i. preparing a plant-based preparation comprising from about 0.5 to 15% by weight of dissolved plant proteins into water;
ii. inoculating said plant-based solution with bacterial strain according to any one of claims **1** to **4** at a concentration of about 10⁶ to 10⁸ CFU / mL substrate;
iii. fermenting the inoculated plant-based solution obtained at step iii), by incubation at a temperature from about 25 to 39^{°}C for about 0.5 to 24 hours;
iv. obtaining a said fermented plant-based product.
